# EUROPÄISCHE PATENTSCHRIFT

(11) **EP 2 999 492 B1**
(45) Veröffentlichungstag und Bekanntmachung des Hinweises auf die Patenterteilung: **08.04.2020**
(21) Anmeldenummer: 14729246.0
(22) Anmeldetag: 23.05.2014
(51) Int. Cl.: A61L 27/10, A61L 27/50, A61L 27/56

(54) **IMPLANTAT AUS KERAMIK MIT PORENKANÄLEN**
IMPLANT CONSISTING OF CERAMICS, COMPRISING PORE CHANNELS
IMPLANT EN CÉRAMIQUE COMPRENANT DES CANAUX DE PORES

(30) Priorität: 23.05.2013 DE 102013209584
(43) Veröffentlichungstag der Anmeldung: 30.03.2016
(73) Patentinhaber: CeramTec GmbH, 73207 Plochingen (DE)
(72) Erfinder: KUNTZ, Meinhard, 73733 Esslingen (DE); WECKER, Heinrich, 90542 Eckental (DE); KELNBERGER, Alfons, 90552 Röthenbach (DE); FRIEDERICH, Kilian, 73207 Plochingen (DE); BIOTTEAU, Katia, 28630 Barjouville (FR); MESSMER, Moritz, 70372 Stuttgart (DE)
(74) Vertreter: Fehrenbacher, Eckhard Anton
(86) Internationale Anmeldenummer: PCT/EP2014/060691
(87) Internationale Veröffentlichungsnummer: WO 2014/187969

(56) Entgegenhaltungen:
- DE-A1- 19 857 958
- DE-A1- 19 940 717
- DE-A1-102008 001 402
- US-A- 4 318 874
- US-A1- 2002 169 066
- US-A1- 2003 171 822
- US-A1- 2008 213 611
- US-A1- 2011 313 538

## Beschreibung

Gegenstand der vorliegenden Erfindung ist ein Implantat das aus einer festen, lasttragenden, keramischen Hülle, einer keramischen, porösen Füllung und die Füllung durchziehenden Porenkanälen besteht. Dieses Implantat kann als Spacer Verwendung finden.

Poröse Materialien sind für das Anhaften und Anwachsen von neuem Zellgewebe wie Knochenzellen besonders geeignet. Die poröse Komponente sollte aus einem belastbaren, biokompatiblen Material bestehen, das biologische Zellanhaftungen, die Vermehrung von Zellen und deren biologische Funktion fördert. Das setzt voraus, dass eine ausreichende Versorgung mit biologischen Flüssigkeiten und Nährstoffen am Ort der Zellneubildung gewährleistet ist. Aus diesen Gründen sollten die porösen Komponenten einen perkolierenden Porenvolumenanteil besitzen.

Darüber hinaus sollte die Porengröße im Größenbereich der neugebildeten Zellen liegen. Das Implantat muss außerdem ausreichend stabil sein, um die Operation und die anfängliche Belastung in vivo ohne Beschädigung überstehen zu können.

In der Vergangenheit wurden verschiedene Techniken zur Erzeugung von porösen Bauteilen und insbesondere keramischen Bauteilen mit einer definierten Porosität verwendet. Bislang existiert jedoch kein Verfahren, mit dem kostengünstig in kurzer Zeit große Stückzahlen dieser Bauteile in zuverlässiger Bauweise erzeugt werden können.

US2011313538 beschreibt Scaffolds/Implantate mit äußerer (Cortex-)Schale und (Bälkchenknochen-)Kern, mit interkonnektierenden Mikroporen und sekundären Mikrokanälen in der Schale und/oder im Kern.

Aufgabe der vorliegenden Erfindung ist es, ein Implantat bereitzustellen, das das formschlüssige An- bzw. Einwachsen von Zellen in besonderer Weise fördert, sowie ein Verfahren bereitzustellen mit dem kostengünstig in kurzer Zeit große Stückzahlen dieses Implantats gefertigt werden können. Gleichzeitig soll das Implantat eine im Vergleich zu Stand der Technik erheblich verbesserte Primär- und Sekundärfestigkeit gewährleisten.

Die Erfindung wird durch die Ansprüche definiert.

Die erfindungsgemäße Lösung sieht ein Implantat vor, das wie folgt aufgebaut ist und aus Keramik besteht:
- Die Hülle besteht aus einem lasttragenden, keramischen, hochfesten, dichten, biokompatiblen Material. Die Hülle wird so gestaltet, dass sie auch ohne Füllung die auf das Implantat wirkenden äußeren Kräfte vollständig aufnehmen kann. Der Porenvolumengehalt der Hülle liegt unter 3 Vol.-%, bevorzugt unter 2 Vol.-% und besonders bevorzugt unter 1 Vol.-%.
- Das Implantat enthält eine Füllung aus keramischem, porösen Material, wobei die Porosität in einer zufälligen Form vorliegt, einen Volumengehalt von 15 - 90 Vol.-%, bevorzugt 20 - 70 Vol.-% und besonders bevorzugt 25 - 50 Vol.-% einnimmt und ein perkolierendes, das heißt für Flüssigkeiten durchlässiges verbundenes Netzwerk bildet. Die Porengröße in dem porösen Material der Füllung liegt im Bereich von 2 bis 400 µm, bevorzugt in der Größenordnung von 5 bis 200 µm.
- Die poröse Füllung soll zwar ein durchlässiges, perkolierendes Porennetzwerk aufweisen. Gleichzeitig jedoch wird Porenstruktur und Porenvolumengehalt bevorzugt so realisiert, dass ein Material mit vergleichsweise hoher Restfestigkeit entsteht. Die Druckfestigkeit der porösen Füllung beträgt bevorzugt > 250 MPa, besonders bevorzugt > 500 MPa. Dadurch steht dem anwachsenden Knochen im Gegensatz zu schwamm- oder schaumartigen Werkstoffen ein vergleichsweise festes und bruchsicheres Substrat zur Verfügung. Erfindungsgemäß wird dadurch eine hohe Primär- und Sekundärfestigkeit, d.h. Festigkeit während des Einwachsvorgangs und nach dem Einwachsvorgang, des Implantats erreicht. Aufgrund dieses Konzepts wird in dieser Erfindung der Porenvolumengehalt der Füllung besonders bevorzugt auf maximal 50% begrenzt.
- Die poröse Füllung des Implantats wird darüber hinaus von definierten, gerichteten Porenkanälen durchzogen, die in Vorzugsrichtung über die gesamte Höhe und entlang der Höhe des Implantats in waagerechter Ausrichtung zur Hülle ausgerichtet sind. Die Kanäle verlaufen geradlinig. Der Durchmesser der Porenkanäle liegt zwischen 0,1 und 2 mm, je nach der Größe der Zellen, die einwachsen sollen. Bevorzugt liegt der Durchmesser der Porenkanäle zwischen 0,3 und 1 mm.
- Die Porenkanäle sind dazu eingerichtet, im Flüssigkeitsaustausch mit der porösen Füllung stehen zu können. Dies kann beispielsweise dadurch erreicht werden, dass die Porenkanäle durch die Porositätsstruktur der Füllung hindurch schneiden und durch dadurch erzeugte Öffnungen in der Wandung der Porenkanäle mit diesen in hydraulischer Verbindung stehen.
- Die Wandung der Porenkanäle wird so gestaltet, dass einerseits ein Flüssigkeitszutritt durch die poröse Keramikfüllung möglich ist und weiterhin eine stark ausgeprägte Oberflächenrauheit mit einer mittleren Rautiefe von Rz 10 - 250 µm, bevorzugt Rz 20 - 200 µm (bestimmt nach DIN EN ISO 4287) vorliegt. Für den einwachsenden Knochen wird somit eine intensive Verzahnung mit der Struktur der Wände der Porenkanäle ermöglicht.

Biokompatible Materialien, die für das Implantat verwendet werden können, umfassen Keramikmaterialien wie Oxid- und Nitridkeramiken.

Das Implantat wird aus einer Keramik gefertigt.

Durch die erfindungsgemäße Lösung wächst der Knochen bevorzugt in die Porenkanäle hinein und zwar im Idealfall von beiden Seiten der Kanäle mit schlussendlicher Verbindung in der Mitte. Für die Verankerung des Implantats im Knochen genügt es aber auch, wenn der Knochen nur ein Stück weit in die Kanäle einwächst, beispielsweise etwa 1 mm tief.

Demgegenüber findet in der porösen Füllung eher kein oder nur ein sehr geringes Einwachsen statt. Die Porengröße ist zu gering, um eine Neubildung von Knochenzellen zu ermöglichen. Aufgrund der vorzugsweise hydrophilen Beschaffenheit des Keramikmaterials wird jedoch die perkolierende Porosität rasch von körpereigenen Medien, beispielsweise Synovialflüssigkeit, vollständig ausgefüllt. Diese trägt zur optimalen Versorgung der sich neu bildenden Knochenzellen in den gerichteten Porenkanälen bei, da die Porenkanäle mit der Porosität der Füllung in hydraulischer Verbindung stehen.

Durch die erfindungsgemäße Verwendung einer porösen Keramik mit perkolierender Porosität kann über Kapillarkräfte Nährmedium transportiert werden. Die anwachsenden Knochenzellen werden somit in idealer Weise mit Nährstoffen versorgt. Die Porosität der Füllung und damit insbesondere der Innenwände der durchgehenden Porenkanäle können erfindungsgemäß so gestaltet werden, dass eine raue Oberfläche mit idealen Bedingungen für das Knochenanwachsen erreicht wird.

Die hydraulische Verbindung zwischen den Porenkanälen und der Porosität der Füllung ermöglicht also vorteilhaft die Versorgung der einwachsenden Knochenzellen am Ort ihrer Bildung. Das bedeutet, dass auch Knochenzellen, die schon weit in das Implantat hineingewachsen sind, noch optimal versorgt werden können. Dies ist bei aus dem Stand der Technik bekannten porösen Materialien häufig nicht der Fall, weil keine unabhängigen Flüssigkeitskommunikationswege zur Verfügung stehen.

Damit können erfindungsgemäß bis zu vier Funktionen gleichzeitig erfüllt werden:
1. Die Hülle sorgt für Festigkeit.
2. Die Kanäle bieten eine optimale Größe für das Knocheneinwachsen.
3. Die perkolierende poröse Keramik ist durchlässig für körpereigene Medien und unterstützt somit die Nährstoffversorgung der in den Porenkanälen anwachsenden Knochenzellen.
4. Die günstige Oberflächentopographie der Kanalwände ermöglicht eine formschlüssige Verankerung des Bauteils beim Einwachsen der Knochenzellen.

Hergestellt wird das erfindungsgemäße Implantat bevorzugt mittels eines keramischen Zwei-Komponenten-Spritzguss. Dabei werden zwei plastifizierbare Gemische aus Keramikpulver und wachsartiger Polymerzubereitung, sogenannte Feedstocks, bereitgestellt. Ein erster Feedstock umfasst das Gemisch aus Keramikpulver und Polymerzubereitung, während der zweite Feedstock zusätzlich noch Porenbildner umfasst.

Der erste Feedstock wird so aufbereitet, dass nach Formgebung, Entbinderung und Sinterung ein fester, dichter Keramikkörper entsteht. Dieser bildet im späteren Implantat die lasttragende Hülle.

Der zweite Feedstock besteht ebenfalls aus Keramikpulver und wachsartiger Polymerzubereitung. Zusätzlich werden hier jedoch noch ausbrennbare organische oder anorganische Porenbildner hinzugemischt ("Fugitive Spacers"). Nach Formgebung, Entbinderung und Sinterung entsteht ein poröser Keramikkörper mit perkolierendem Porennetzwerk, der im Implantat die poröse Füllung bildet. Das Keramikpulver für den zweiten Feedstock wird so gewählt, dass sich bei der Sinterung ein fester Verbund mit der dichten Keramik des ersten Feedstocks ausbildet. Dabei kann eine artgleiche Keramik vorteilhaft sein, ist jedoch nicht zwingend erforderlich. Das bedeutet, dass für den ersten und den zweiten Feedstock das gleiche Gemisch aus Keramikpulver oder ein Gemisch aus einem anderen Keramikpulver verwendet werden kann, wobei die Verwendung des gleichen Gemischs bevorzugt ist.

Bevorzugt können für das Implantat Oxidkeramiken Verwendung finden, besonders bevorzugt sind ZTA (zirconia toughened alumina), ATZ (alumina toughened zirconia), Zirkonoxidbasierte Verbundwerkstoffe mit sonstigen Verstärkungskomponenten oder TZP (tetragonal zirconia polycrystal). Diese Keramiken sind unter anderem deshalb vorteilhaft, weil sie hydrophil sind und optimale Bedingungen für das Eindringen von Körperflüssigkeiten in den Porenraum bieten. Im Rahmen der Erfindung liegen aber auch biokompatible Nichtoxid-Keramiken wie z.B. Siliziumnitrid.

Unter einer "artgleichen Keramik" wird im Rahmen dieser Erfindung eine Keramik verstanden, die aus den gleichen keramischen Grundkomponenten besteht. Insbesondere bevorzugt ist auch das Mengenverhältnis der keramischen Grundkomponenten im Wesentlichen, d.h. in Grenzen von +/- 10 Vol.-% pro Anteil, gleich.

Die beiden Elemente des Implantats werden also aus verschiedenen Feedstocks in einem Arbeitsgang in eine Form eingespritzt, wodurch die Elemente physisch in engem Kontakt zueinander stehen. Dieser Verbund wird anschließend entbindert und gesintert. Es entsteht ein monolithischer Körper, der eine feste, lasttragende Hülle und eine fest darin verbundene poröse Füllung aufweist.

Als Porenbildner kommen grundsätzlich alle Stoffe infrage, die eine ausreichende Form- und Temperaturbeständigkeit für den 2-Komponenten-Spritzguss von Keramikmaterialien aufweisen. Natürlich müssen die Porenbildner, vorzugsweise rückstandsfrei, bei Sintertemperaturen der Keramik ausbrennbar sein.

Die Formbeständigkeit der Porenbildner während des Herstellverfahrens ist wesentlich. Die Poren sollen fluidgängig und perkolierend sein, so dass durch den Spritzguss stark deformierte Porenbildner nicht zu tolerieren sind. Weiterhin dürfen die Porenbildner bzw. die durch sie erzeugten Poren im Gesamtverfahren einschließlich des Sinterns nicht unkontrolliert schrumpfen.

Bevorzugte Porenbildner im Rahmen dieser Erfindung und ohne Beschränkung der Allgemeinheit sind: Maisgries, Melaminharz-Partikel, Polyamid-Partikel, Kohlenstoff-Partikel, glasige Kohlenstoffpartikel, Kohlenstoff-Fasern, Mohnsamen, Getreidemehl, insbesondere Weizenmehl und/oder Kartoffelstärke. Natürlich können auch Kombinationen unterschiedlicher Porenbildner in einem Feedstock Verwendung finden, z.B. sphärische zusammen mit faserigen Partikeln.

Die Porenkanäle können durch die technologische Ausgestaltung des Spritzgusswerkzeugs und/oder durch Nachbearbeitung des Grünkörpers oder des fertig gesinterten Körpers hergestellt werden.

Zur Ausgestaltung des Spritzgusswerkzeugs wird dieses mit stiftartigen Komponenten versehen, die nach der Entformung im Grünkörper kanalartige Strukturen hinterlassen. Die Stifte können zusätzlich mit einer Oberflächenstruktur versehen werden, die sich beim Entformen entsprechend auf der Oberfläche des Grünkörpers abbildet und somit die gewünschte raue Struktur hinterlässt.

Die Porenkanäle können auch durch das gerichtete Einbringen von ausbrennbaren Stäbchen, die wie die oben beschriebenen Porenbildner beim Sintern ausgebrannt werden, erzeugt werden. Die Stäbchen können beispielweise aus einem Polymermaterial bestehen.

Ein so hergestelltes Implantat kann beispielsweise als Spacer, d.h. als Wirbelsäulen-Implantat, beispielsweise nach einer Bandscheibenektomie, Verwendung finden.

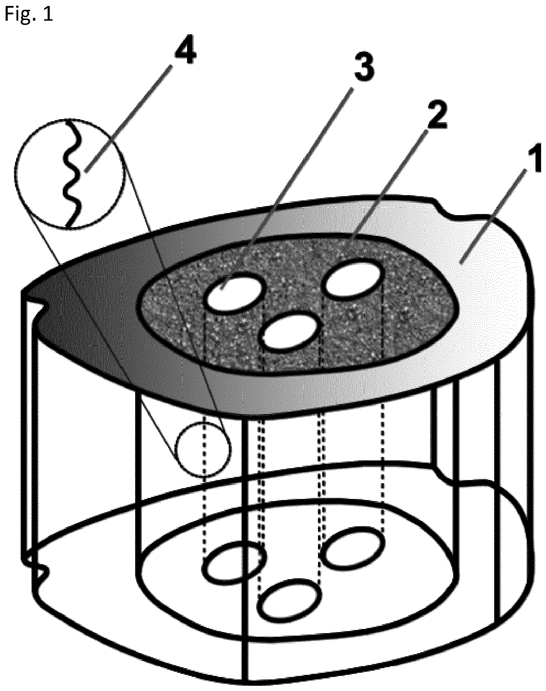

Im Folgenden wird die Erfindung anhand von Beispielen näher erläutert. Figur 1 zeigt eine schematische Ausführungsform eines erfindungsgemäßen Implantats. Das Implantat weist eine feste, lasttragende Hülle 1 auf, die eine poröse Füllung 2 lateral umschließt. An Ober- und Unterseite des Implantats liegt die poröse Füllung 2 frei. Die lasttragenden Hülle hat einen Porenvolumenanteil von weniger als 1 Vol.-%.

Die poröse Füllung 2 weist einen Porenvolumenanteil auf, der zwischen 15 und 90 Vol.-% liegt. Die Angabe des Volumenanteils betrifft hier nur das poröse Material selbst, ohne Berücksichtigung der Porenkanäle. Die Porosität der Füllung ist perkolierend, d.h. die Poren stehen miteinander zumindest zum Teil, bevorzugt überwiegend in Verbindung und erlauben das Eindringen und die Weiterleitung und/oder das Zirkulieren von Körperflüssigkeiten. Anders ausgedrückt bildet diese Porosität ein zusammenhängendes Netzwerk, das sich mit Flüssigkeit füllen kann. Die Poren weisen bevorzugt Durchmesser in einem Bereich zwischen 5 und 200 µm auf.

Die poröse Füllung wird darüber hinaus von durchgängigen Porenkanälen 3 durchzogen, die in dieser Ausführungsform durch die Ober- und Unterseite des Implantats mit der Umgebung in Kontakt stehen. Im implantierten Zustand erlauben die Porenkanäle das Ein- oder Anwachsen von Knochenmaterial in oder an das Implantat. Die Porenkanäle 3 stehen mit dem vorbeschriebenen perkolierenden Porennetzwerk in hydraulischer Verbindung. Durch Kapillarkräfte füllt sich das Porennetzwerk mit Körperflüssigkeiten. Infolge der Verbindung von Porenkanälen und perkolierender Porosität gelangen diese Körperflüssigkeiten auch in das Innere der Porenkanäle, selbst wenn diese schon bereichsweise mit neu gebildetem Knochenmaterial gefüllt sind. Damit ist Versorgung der einwachsenden Knochenzellen mit Nährstoffen in jedem Stadium des Einwachsens sichergestellt.

Das Bezugszeichen 4 zeigt vergrößert einen schematischen Ausschnitt aus einer Wand eines Porenkanals. Die Wand des Porenkanals ist strukturiert oder aufgeraut, so dass sich das neu gebildete Knochenmaterial mit dem Implantat verzahnen kann. Aus dieser Verzahnung resultiert ein verbesserter Halt zwischen Implantat und Knochenmaterial.

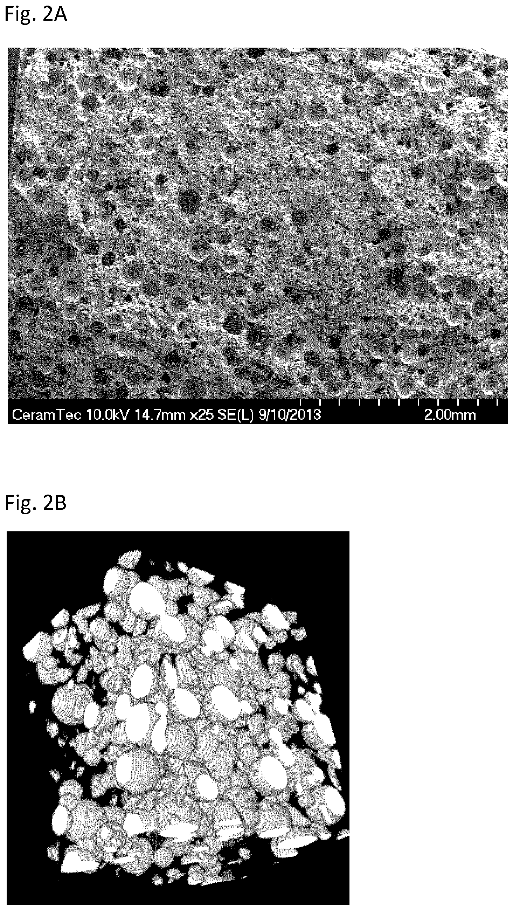

Figur 2A zeigt eine rasterelektronenmikroskopische Aufnahme eines aufgebrochenen Ausschnitts einer erfindungsgemäßen porösen Füllung. Als Werkstoff wurde eine Yttrium-stabilisierte TZP-Keramik verwendet. Die individuellen Porengrößen sind sehr unterschiedlich und liegen in einem Bereich zwischen 20 und 300 µm. Die Porosität wurde durch Ausbrennen von kugelförmigen Kohlenstoff-Partikeln erzeugt.

In Fig. 2B ist eine computertomographische Rekonstruktion des perkolierenden Netzwerkes des in Fig. 2A dargestellten Füllungsmaterials zu sehen.

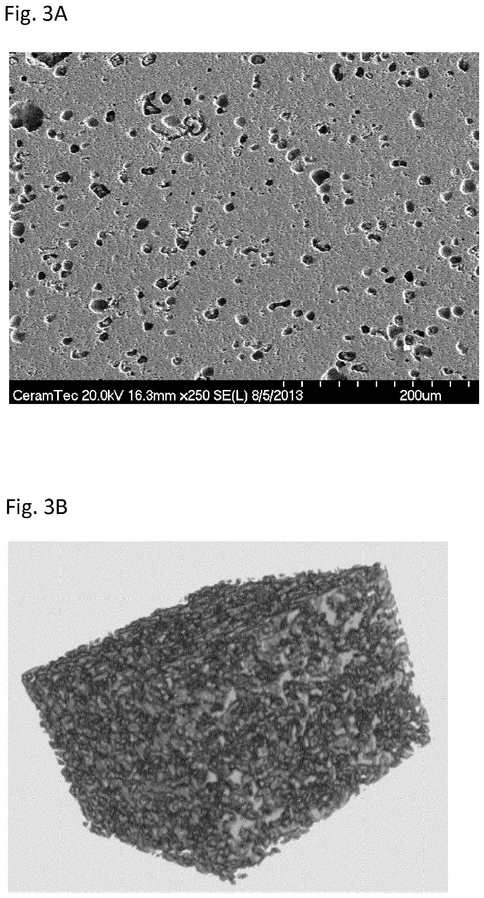

Figur 3A zeigt eine weitere Ausführungsform der porösen Füllung in einer rasterelektronenmikroskopischen Aufnahme eines Anschliffs und die zugehörige computertomographische Rekonstruktion des Porennetzwerks, siehe Fig. 3B. Im Unterschied zu dem in den Figuren 2A und 2B dargestellten Material wurde hier ein feinkörnigerer Porenbildner, nämlich Maisgries mit Korngrößen zwischen 5 und 50µm zur Erzeugung der Porosität verwendet. Auch diese Porosität ist perkolierend, obwohl der Porenvolumenanteil deutlich niedriger bei ca. 25 Vol.-% liegt als in dem in

Fig. 2 dargestellten Beispiel. Als Keramikwerkstoff wurde eine ZTA-Keramik verwendet.

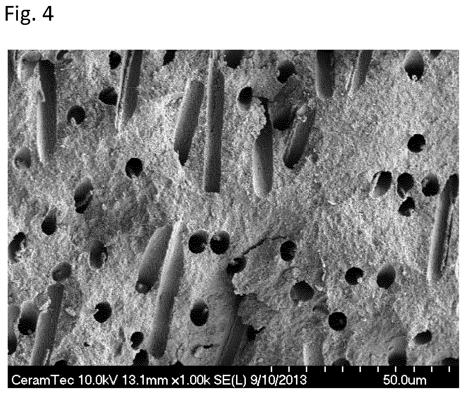

Figur 4 zeigt eine weitere Ausführungsform der porösen Füllung aus einer Y-stabilisierten TZP-Keramik. Als Porenbildner wurden Carbon-Fasern mit einem Durchmesser von 7 µm und einer Länge von 150 µm verwendet.

## Patentansprüche

1. Implantat, umfassend eine feste, lasttragende, keramische Hülle und eine keramische, poröse Füllung, **dadurch gekennzeichnet, dass** die poröse Füllung von gerichteten Porenkanälen, die entlang der Höhe des Implantats in waagerechter Ausrichtung zur Hülle angeordnet sind, durchzogen ist, wobei der Porenvolumenanteil der Hülle unter 3 Vol.-%, bevorzugt unter 2 Vol.-% und besonders bevorzugt unter 1 Vol.-% liegt, und wobei die Wände der Porenkanäle eine Oberflächenrauheit mit einer mittleren Rautiefe von Rz 10 bis 250 µm aufweisen und wobei die Porenkanäle einen Durchmesser von 0,1-2 mm haben und die Porengröße der porösen Füllung im Bereich von 2 bis 400 µm liegt.

2. Implantat nach einem der vorstehenden Ansprüche, **dadurch gekennzeichnet, dass** der Porenvolumenanteil der porösen Füllung zwischen 15 und 90 Vol.-%, bevorzugt zwischen 20 und 70 Vol.-% und insbesondere bevorzugt zwischen 25 und 50 Vol.-% liegt.

3. Implantat nach einem der vorstehenden Ansprüche, **dadurch gekennzeichnet, dass** die Porosität der Füllung perkolierend ist.

4. Implantat nach einem der vorstehenden Ansprüche, **dadurch gekennzeichnet**, die poröse Füllung Porengrößen im Bereich von 5 bis 200 µm aufweist.

5. Implantat nach einem der vorstehenden Ansprüche, **dadurch gekennzeichnet, dass** die Porenkanäle der porösen Füllung einen durchschnittlichen Durchmesser von 0,1 bis 2 mm, bevorzugt von 0,3 bis 1 mm aufweisen.

6. Implantat nach einem der vorstehenden Ansprüche, **dadurch gekennzeichnet, dass** die Wände der Porenkanäle eine Oberflächenrauheit mit einer mittleren Rautiefe von Rz 20 bis 200 µm aufweisen.

7. Implantat nach einem der vorstehenden Ansprüche, **dadurch gekennzeichnet, dass** die Porenkanäle mit der Porosität der Füllung in hydraulischem Kontakt stehen.

8. Verfahren zur Herstellung eines keramischen Implantats gemäß einem der vorstehenden Ansprüche, **dadurch gekennzeichnet, dass** ein keramisches Zwei-Komponenten-Spritzguss-Verfahren angewendet wird.

9. Verfahren nach Anspruch 8, **dadurch gekennzeichnet, dass** aus zumindest einem Gemisch aus Keramikpulver und zumindest einer wachsartigen Polymerzubereitung ein erster und ein zweiter Feedstock gebildet werden, wobei der zweite Feedstock zusätzlich ausbrennbare Porenbildner umfasst.

10. Verfahren nach Anspruch 9, **dadurch gekennzeichnet, dass** für den ersten und den zweiten Feedstock das gleiche Gemisch aus Keramikpulver oder unterschiedliche Gemische aus Keramikpulver verwendet werden.

11. Verfahren nach einem der Ansprüche 8 bis 10, **dadurch gekennzeichnet, dass** die Porenbildner aus einem oder mehreren der Gruppe Maisgries, Melaminharz-Partikel, Polyamid-Partikel, Kohlenstoff-Partikel, glasige Kohlenstoffpartikel, Kohlenstoff-Fasern, Mohnsamen, Getreidemehl, insbesondere Weizenmehl und/oder Kartoffelstärke ausgewählt werden.

12. Verfahren nach einem der Ansprüche 8 bis 11, **dadurch gekennzeichnet, dass** ein Spritzguss-Werkzeug mit stiftartigen Komponenten verwendet wird, so dass beim Spritzgießen im Material des zweiten Feedstocks durchgehende Porenkanäle erzeugt werden.

13. Verfahren nacheinem der Ansprüche 8 bis 11, **dadurch gekennzeichnet, dass** durch Nachbearbeitung des Grünkörpers oder des fertig gesinterten keramischen Implantats durchgehende Porenkanäle im Material des zweiten Feedstocks erzeugt werden.

## Claims

1. Implant, comprising a solid, load-bearing, ceramic shell and a ceramic, porous filling, **characterized in that** the porous filling is permeated by directed pore channels which are arranged along the height of the implant in a horizontal orientation with respect to the shell, the volume fraction of pores in the shell being less than 3 vol.%, preferably less than 2 vol.% and particularly preferably less than 1 vol.%, and the walls of the pore channels having a surface roughness having an average roughness depth of Rz 10 to 250 µm and the pore channels having a diameter of 0.1-2 mm and the pore size of the porous filling being in the range of 2 to 400 µm.

2. Implant according to the preceding claim, **characterized in that** the volume fraction of pores in the porous filling is between 15 and 90 vol.%, preferably between 20 and 70 vol.% and particularly preferably between 25 and 50 vol.%.

3. Implant according to either of the preceding claims, **characterized in that** the porosity of the filling is percolating.

4. Implant according to any of the preceding claims, **characterized in that** the porous filling has pore sizes in the range of 5 to 200 µm.

5. Implant according to any of the preceding claims, **characterized in that** the pore channels of the porous filling have an average diameter of 0.1 to 2 mm, preferably of 0.3 to 1 mm.

6. Implant according to any of the preceding claims, **characterized in that** the walls of the pore channels have a surface roughness having an average roughness depth of Rz 20 to 200 µm.

7. Implant according to any of the preceding claims, **characterized in that** the pore channels are in hydraulic contact with the porosity of the filling.

8. Method for producing a ceramic implant according to any of the preceding claims, **characterized in that** a ceramic two-component injection molding method is used.

9. Method according to claim 8, **characterized in that** a first and a second feedstock are formed from at least one mixture of ceramic powder and at least one wax-like polymer preparation, the second feedstock additionally comprising combustible pore forming materials.

10. Method according to claim 9, **characterized in that** the same mixture of ceramic powder or different mixtures of ceramic powder are used for the first and the second feedstock.

11. Method according to any of claims 8 to 10, **characterized in that** the pore forming materials are selected from one or more of the group consisting of corn meal, melamine resin particles, polyamide particles, carbon particles, glassy carbon particles, carbon fibers, poppy seeds, and cereal flour, in particular wheat flour and/or potato starch.

12. Method according to any of claims 8 to 11, **characterized in that** an injection molding tool comprising pin-like components is used, such that continuous pore channels are created in the material of the second feedstock during injection molding.

13. Method according to any of claims 8 to 11, **characterized in that** continuous pore channels are created in the material of the second feedstock by reworking the green body or the finished sintered ceramic implant.

## Revendications

1. Implant comprenant une enveloppe en céramique solide et porteuse de charge et un remplissage poreux en céramique, **caractérisé en ce que** le remplissage poreux est traversé par des canaux de pores se faisant face, lesquels sont disposés le long de la hauteur de l'implant selon une orientation horizontale par rapport à l'enveloppe, la proportion en volume de pores de l'enveloppe étant inférieure à 3 % en volume, de préférence inférieure à 2 % en volume et de manière particulièrement préférée inférieure à 1 % en volume, et les parois des canaux de pores présentant une rugosité de surface avec une profondeur de rugosité moyenne de Rz = 10 à 250 µm, et les canaux de pores ayant un diamètre de 0,1 à 2 mm et la taille de pore du remplissage poreux étant comprise entre 2 et 400 µm.

2. Implant selon l'une des revendications précédentes, **caractérisé en ce que** la proportion en volume de pores du remplissage poreux est comprise entre 15 et 90 % en volume, de préférence entre 20 et 70 % en volume et de manière particulièrement préférée entre 25 et 50 % en volume.

3. Implant selon l'une des revendications précédentes, **caractérisé en ce que** la porosité du remplissage est percolante.

4. Implant selon l'une des revendications précédentes, **caractérisé en ce que** le remplissage poreux a des tailles de pores comprises entre 5 et 200 µm.

5. Implant selon l'une des revendications précédentes, **caractérisé en ce que** les canaux de pores du remplissage poreux ont un diamètre moyen de 0,1 à 2 mm, de préférence de 0,3 à 1 mm.

6. Implant selon l'une des revendications précédentes, **caractérisé en ce que** les parois des canaux de pores présentent une rugosité de surface avec une profondeur de rugosité moyenne de Rz = 20 à 200 µm.

7. Implant selon l'une des revendications précédentes, **caractérisé en ce que** les canaux de pores sont en contact hydraulique avec la porosité du remplissage.

8. Procédé de fabrication d'un implant en céramique selon l'une des revendications précédentes, **caractérisé en ce que** l'on utilise un procédé de moulage par injection de céramique à deux composants.

9. Procédé selon la revendication 8, **caractérisé en ce qu'**une première et une seconde matières premières sont constituées d'au moins un mélange de poudre de céramique et d'au moins une composition de polymère cireuse, la seconde matière première comprenant en outre des agents porogènes combustibles.

10. Procédé selon la revendication 9, **caractérisé en ce que** le même mélange de poudre de céramique ou différents mélanges de poudre de céramique sont utilisés pour la première et la seconde matières premières.

11. Procédé selon l'une des revendications 8 à 10, **caractérisé en ce que** les agents porogènes sont choisis parmi un ou plusieurs des groupes constitués par des semoules de maïs, des particules de résine de mélamine, des particules de polyamide, des particules de carbone, des particules de carbone vitreux, des fibres de carbone, des graines de pavot, de la farine de céréales, en particulier de la farine de blé et/ou de l'amidon de pomme de terre.

12. Procédé selon l'une des revendications 8 à 11, **caractérisé en ce que** l'on utilise un outil de moulage par injection présentant des composants en forme de broches, de sorte que, lors du moulage par injection, des canaux de pores continus sont produits dans le matériau de la seconde matière première.

13. Procédé selon l'une des revendications 8 à 11, **caractérisé en ce que** des canaux de pores continus sont produits dans le matériau de la seconde matière première par le traitement ultérieur du corps vert ou de l'implant en céramique à frittage fini.
